(19) Europäisches Patentamt
European Patent Office
Office européen des brevets

(11) Publication number: **0 302 183 A2**

(12) **EUROPEAN PATENT APPLICATION**

(21) Application number: **88106923.1**

(51) Int. Cl.[4]: **A01N 43/86 , A01H 1/02**

(22) Date of filing: **29.04.88**

(30) Priority: **05.08.87 US 47759**

(43) Date of publication of application:
**08.02.89 Bulletin 89/06**

(84) Designated Contracting States:
**AT BE CH DE ES FR GB GR IT LI LU NL SE**

(71) Applicant: **CHEMBRED, INC.**

**Princeton New Jersey 08540(US)**

(72) Inventor: **Olvey, James M.**
**6703 S. Mitchell Drive**
**Tempe Arizona 85283(US)**

(74) Representative: **Wächtershäuser, Günter, Dr.**
**Tal 29**
**D-8000 München 2(DE)**

(54) **Hybrid corn seed and method.**

(57) A method for hybridizing corn (zea mays) by crossing two corn varieties by rendering a female parent male sterile with an isothiazole plant growth regulator and then pollinating the male sterile female parent with pollen from a second variety.

EP 0 302 183 A2

# HYBRID CORN SEED AND METHOD

## FIELD OF THE INVENTION

This invention relates to a method for hybridizing corn by crossing two corn varieties through the use of a chemical application technique to provide a male sterile female parent variety having fertile female parts that can be pollinated with male fertile pollen of a second variety.

## BACKGROUND OF THE INVENTION

Zea mays or corn is an agronomically important crop in many countries of the word and is extremely commercially important in the United States. Corn is used for feed, for food and for industrial purposes.

Although inbred lines which have been developed by various breeders in corn research are not grown as a commercial crop, they are extremely important because they are employed to produce first generation ($F_1$) hybrids by the hybridization of, for example, two inbred lines as parents. As a result of the crossing of the two inbred lines, hybrid vigor or heterosis arises and the hybrid plants produced generally have markedly improved yields, better stalks, better roots, better uniformly and better insect and disease resistance. Further, as a result of self-pollination of $F_1$ hybrid plants or cross-pollination of $F_1$ hybrid plants, a second generation ($F_2$) hybrid occurs due to the self-pollination or cross-pollination of $F_1$ hybrid plants. The $F_2$ hybrid plant, and seed produced thereby, has characteristics which are less desirable than those of the $F_1$ hybrid, for example, lower yields, and expression of undesirable genetic traits results. Due to this reduced performance, seed from $F_1$ hybrids which produces less advantageous second generation $F_2$ hybrids is generally not saved by farmers. Rather, new hybrid seed produced by crossing the originally selected parents to produce the first generation hybrid ($F_1$) seed is purchased from commercial seed companies by farmers each year for their planting.

Currently, male sterilization by emasculation of the female parent of the hybrid is generally performed by the expensive route of hand detasseling. Alternate routes for detasseling to provide male sterilization involve machine detasseling, chemical sterilization or cytoplasmic techniques.

Because of the large volume of hybrid corn production and the advantage of using early generation hybrid seed, such as $F_1$, or $F_2$ seeds, substantial cost savings are inherent with the use of a highly efficient chemical male sterilization method as provided by the present invention.

## SUMMARY OF THE INVENTION

The method of this invention is defined as the method for rendering a first variety corn plant male sterile to provide a corn plant that is male sterile with female fertile parts that can be hybridized with a second variety corn plant by pollination of the fertile female parts with pollen from the second variety corn plant during the pollination period, the pollinated first plant female parts then being capable of maturing to form a corn plant having hybrid corn seeds, comprising:

(a) contacting said first variety corn plant prior to formation of first plant female parts and male fertile parts with a male part sterilizing effective amount of an isothiazole plant growth regulator that is less than a female part sterilizing amount of regulator;

(b) maintaining said male part sterilizing effective amount of regulator in (a) in contact with said first plant throughout a duration of the pollination period of the growing season of the first plant sufficient to provide a corn plant that is male sterile with female fertile plant parts.

Preferably, the effective amount of plant growth regulator is within the range of about 0.05 to about 2.0 pounds per acre and said plant growth regulator is applied at least five days prior to said formation of first plant female parts and male fertile parts.

The male sterilizing effective amount of the plant growth regulator is maintained at least until set of corn kernals has occurred within the female parts.

The plant growth regulator is applied in sufficient time prior to the formation of male fertile parts to render said male parts sterile prior to the female parts maturing to reach a period of female fertility.

The preferred isothiazole plant growth regulator is a compound selected from the group of compounds consisting essentially of potassium 3,4-dichloro-5-isothiazolecarboxylate; a 3,4-dichloro-5-isothiazolecarboxylate; cyclohexyl 3,4-dichloro-5-isothiazolecarboxylate; phenyl 3,4-dichloroisothiazolecarboxylate; p-chlorophenyl a 3,4-dichloro-5-isothiazolecarboxylate; p-tolyl 3,4-dichloro-5-isothiazolecarboxylate; p-anisyl 3,4-dichloro-5-isothiazolecarboxylate; 2-naphthyl 3,4-dichloro-5-isothiazolecarboxylate; poly(ethylene glycol)200 bis (3,4-dichloro-5-isothiazolecarboxylate; poly(ethylene glycol)1000 bis(3,4-dichloro-5-isothiazolecarboxylate); 3,4-dichloro-5-isothiazolecarboxanilide; 4-methylcyclohexyl 3,4-dichloro-5-isothiazolecarboxylate; 3,3,5-trimethylcyclohexyl 3,4-dichloro-5-isothiazolecarboxylate; 2-sec-butyl-4-6-dinitrophenyl 3,4-dichloro-5-isothiazolecarboxylate; 3-trifluoromethylphenyl 3,4-dichloro-5-isothiazolecarboxylate; 2,6-dichloro-4-(fluorosulfonyl)phenyl 3,4-dichloro-5-isothiazolecarboxylate; S-(3-propylthio)propyl 3,4-dichloro-5-isothiazolecarbothioate; 3,4-dichloro-2′,4′-dinitro-5-isothiazolecarboxanilide; N-(2-pyridyl)-3,4-dichloro-5-isothiazolecarboxamide; 4-chloro-2-fluorosulfonylphenyl 3,4-dichloro-5-isothiazole-carboxylate; allyl 3,4-dichloro-5-isothiazolecarboxylate; 2-(p-methoxyphenoxy)ethyl 3,4-dichloro-5-isothiazolecarboxylate; 2-(p-chlorophenylthio)ethyl 3,4-dichloro-5-isothiazolecarboxylate; and p-bromphenyl 3,4-dichloro-5-isothiazolecarboxylate.

Also included within the invention are the first variety male sterile corn plants having female fertile parts of the above methods.

The hybridizing method of this invention includes the methods as above defined wherein the female fertile first plant parts are exposed to male fertile pollen from said second variety corn plant to provide pollinated first plant female parts, said pollinated female parts then maturing during the remainder of the growing season to provide a corn plant having hybrid corn seeds.

It is preferred that the female fertile first plant parts be exposed to male fertile pollen from said second variety corn plant to provide pollinated first plant female parts, said pollinated female parts then maturing during the remainder of the growing season to provide a corn plant having hybrid corn seeds.

Also within the scope of theinvention are corn seeds, corn plants, and plant tissue of the above hybridizing methods.

Hybrid corn seed per se containing an isothiazole compound, preferably less than 10 ppm by weight of seed, are also within the scope of the invention.

## DETAILED DESCRIPTION OF THE INVENTION

The isothiazole plant growth regulators such as 3,4-dichloro-isothiazole-5-carboxilic are useful, in general, acid as plant growth regulators.

There are numerous derivatives of the basic 3,4-dichloro-isothiazole nucleus having various moieties connected through the number 5 position. In practicing the present invention it is postulated that similar results are obtainable with these various derivatives as the primary active portion of the compound is the basis 3,4-dichloro-isothiazole-5-nucleus, irrespective of what moiety is linked through the five position, insofar as the linked moiety does not substantially interfere with the activity of the basic nucleus.

The method of rendering cotton plants male sterile with an isothiazole plant growth regulator and then hybridizing such cotton plants with a second variety is known in the art.

The preferred plant growth regulator in practicing my present invention is potassium 3,4-dichloro-5-isothiazolecarboxylate (TD-1123) as it is quite soluble in water and relatively inert and non-toxic to humans under the conditions of use in the instant context.

As utilized in this specification the male part of the corn plant refers to the tassel wherein the corn pollen is generated and held until release. The reference to male sterility is intended to include the situation where no tassels are developed and/or such tassels are otherwise undeveloped in some particular and the pollen produced is not fertile and not capable of inducing set in the female part of the plant.

The female part of the plant refers to the area of the plant where the corn ears, comprising a cob with kernals are produced. This is the area where silks first appear and where male fertile pollen must be applied at the appropriate time to induce pollination and set. Female fertility means that the female part when exposed to male fertile pollen from the same plant or an adjacent plant is capable of being pollinated.

In practicing the present invention it is most desirable to select an appropriate female parent and plant such a parent in a row under normal conditions (referred to hereinafter as the first plant variety). Similarly an appropriate male parent is selected and planted in a row adjacent to said first variety (the male parent being hereinafter referred to as the second plant variety). At the appropriate time prior to maturity of the male part of the plant the isothiazole plant growth regulator is applied to the first plant variety to render the first plant variety male sterile.

The amount of isothiazole compound applied to the first variety plant is preselected and will vary depending upon the specific variety and climatic conditions. However, under most conditions if the time is appropriate, complete male sterility without adversely affecting female fertility can be obtained with a single (TD-1123) application. The most practical method for applying the isothiazole plant growth regulator to the plant is to dilute the isothiazole compound with water to provide about 300 to 400 parts per million of (ppm) active per

part water, typically. The diluted active is then sprayed onto each individual first variety corn plant to the point of runoff. Such application to the first variety plant is typically made at least five days prior to the male part of the plant forming male fertile pollen. However, this time interval can be established with a high degree of certainty empirically in any given situation as the precise timing will depend upon variety and climatic conditions. The amount of active per acre applied is widely variable and can range anywhere from 0.05 pounds of the isothiazole compound per acre to 2 pounds of isothiazole compound per acre.

A typical sweet corn variety was planted under ideal growing conditions in a greenhouse. Prior to the female part of the corn becoming female fertile and prior to production of male fertile pollen or any tasseling, the plants were individually sprayed to the point of runoff with TD-1123 that had been diluted in water to about 300 ppm. Male sterility in the plant was observed as the tassels where either nonexistent or only slightly developed and no male fertile pollen was produced. The female parts of the plant appeared to be unaffected and to remain female fertile.

The above male sterilized female fertile sweet corn plants can be pollinated with male fertile pollen from a second variety to provide a hybrid corn.

In a second trial a commercial field corn variety was similarly treated with the plant growth regulator (TD-1123) and male sterility was again observed. However, with field corn, the tassels appeared to form, but the pollen that was produced was not capable of creating set or pollinating the female fertile portions or parts of the plant. This was manifested by the observation that, although the female portion formed a cob with shucks and silks at maturity, no kernals were present, indicating a failure to pollinate and set due to incudced male sterility.

The female parts of the above field corn remained female fertile and can be crossed with a second variety merely by treating the female parts with male fertile pollen from a second variety.

Although TD1123 is presently the preferred isothiazole plant growth regulator for the reasons above stated, it is believed that the various derivatives of TD1123 having non-interfering substituents would give similar performance. Included among these derivatives are: potassium 3,4-dichloro-5-isothiazolecarboxylate; a 3,4-dichloro-5-isothiazolecarboxylate; cyclohexyl 3,4-dichloro-5-isothiazolecarboxylate; p-chlorophenyl 3,4-dichloro-5-isothiazolecarboxylate; p-chlorophenyl a 3,4-dichloro-5-isothiazolecarboxylate; p-tolyl 3,4-dichloro-5-isothiazolecarboxylate; p-anisyl 3,4-dichloro-5-isothiazolecarboxylate; 2-naphthyl 3,4-dichloro-5-isothiazolecarboxylate; poly(ethylene glycol)200 bis (3,4-dichloro-5-isothiazolecarboxylate; poly(ethyleneglycol)1000 bis (3,4-dichloro-5-isothiazolecarboxylate); 3,4-dichloro-5-isothiazolecarboxanilide; 4-methylcyclohexyl 3,4-dichloro-5-isothiazolecarboxylate; 3,3,5-trimethylcyclohexyl 3,4-dichloro-5-isothiazolecarboxylate; 2-sec-butyl-4-6-dinitrophenyl 3,4-dichloro-5-isothia zolecarboxylate; 3-trifluoromethylphenyl 3,4-dichloro-5-isothiazolecarboxylate; 2,6-dichloro-4-(fluorosulfonyl)phenyl 3,4-dichloro-5-isothiazolecarboxylate; S-(3-propylthio)propyl 3,4-dichloro-5-isothiazolecarbothioate; 3,4-dichloro-2′,4′-dinitro-5-isothiazolecarboxanilide; N-(2-pyridyl)-3,4-dichloro-5-isothiazolecarboxamide; 4-chloro-2-fluorosulfonylphenyl 3,4-dichloro-5-isothiazole-carboxylate; allyl 3,4-dichloro-5-isothiazolecarboxylate; 2-(p-methoxyphenoxy)ethyl 3,4-dichloro-5-isothiazolecarboxylate; 2-(p-chlorophenylthio)ethyl 3,4-dichloro-5-isothiazolecarboxylate; and p-bromphenyl 3,4-dichloro-5-isothiazolecarboxylate.

## Claims

1. A method for rendering a first variety corn plant male sterile to provide a corn plant that is male sterile with female fertile parts that can be hybridized with a second variety corn plant by pollination of the fertile female parts with pollen from the second variety corn plant during the pollination period, the pollinated first plant female parts then being capable of maturing to form a corn plant having hybrid corn seeds, comprising:

(a) contacting said first variety corn plant prior to formation of first plant female parts and male fertile parts with a male part sterilizing effective amount of an isothiazole plant growth regulator that is less than a female part sterilizing amount of regulator;

b) maintaining said male part sterilizing effective amount of regulator in (a) in contact with said first plant throughout a duration of the pollination period of the growing season of the first plant sufficient to provide a corn plant that is male sterile with female fertile plant parts.

2. The method as in claim 1 wherein in (a) the effective amount of plant growth regulator is within the range of about 0.05 to about 2.0 pounds per acre.

3. The method as defined in claim 1 wherein in (a) the plant growth regulator is applied at least about five days before said formation of first plant female parts and male fertile parts, and in (b) said male sterilizing effective amount of the plant growth regulator is maintained at least until set of corn kernals has occurred within the female parts.

4. The method as defined in any one of claims 1 to 3 wherein the isothiazole plant growth regulator is a compound selected from the group of compounds consisting essentially of potassium 3,4-dichloro-5-isothiazolecarboxylate; a 3,4-dichloro-5-isothiazolecarboxylate; cyclohexyl 3,4-dichloro-5-isothiazolecarboxylate; phenyl 3,4-dichloro-5-isothiazolecarboxylate; p-chlorophenyl a 3,4-dichloro-5-isothiazolecarboxylate; p-tolyl 3,4-dichloro-5-isothiazolecarboxylate; p-anisyl 3,4-dichloro-5-isothiazolecarboxylate; 2-naphthyl 3,4-dichloro-5-isothiazolecarboxylate; poly(ethylene glycol)200 bis (3,4-dichloro-5-isothiazolecarboxylate; poly(ethylene glycol)1000 bis(3,4-dichloro-5-isothiazolecarboxylate; 3,4-dichloro-5-isothiazolecarbox anilide; 4-methylcyclohexyl 3,4-dichloro-5-isothiazolecarboxylate; 3,3,5-trimethylcyclohexyl 3,4-dichloro-5-isothiazolecarboxylate; 2-sec-butyl-4-6-dinitrophenyl 3,4-dichloro-5-isothiazolecarboxylate; 3-trifluoromethylphenyl 3,4-dichloro-5-isothiazolecarboxylate; 2,6-dichloro-4-(fluorosulfonyl)phenyl 3,4-dichloro-5-isothiazolecarboxylate; S-(3-propylthio)propyl 3,4-dichloro-5-isothiazolecarbothioate; 3,4-dichloro-2′,-4′-dinitro-5-isothiazolecarboxanilide; N-(2-pyridyl)-3,4-dichloro-5-isothiazolecarboxamide; 4-chloro-2-fluorosulfonylphenyl 3,4-dichloro-5-isothiazolecarboxylate; allyl 3,4-dichloro-5-isothiazolecarboxylate; 2-(p-methoxyophenoxy)ethyl 3,4-dichloro-5-isothiazolecarboxylate; 2-(p-chlorophenylthio)ethyl 3,4-dichloro-5-isothiazolecarboxylate; and p-bromphenyl 3,4-dichloro-5-isothiazolecarboxylate.

5. The first variety male sterile corn plant having female fertile parts of any one of claims 1 to 4.

6. The method of any one of claims 1 to 3 wherein the plant growth regulator is a compound having the formula:

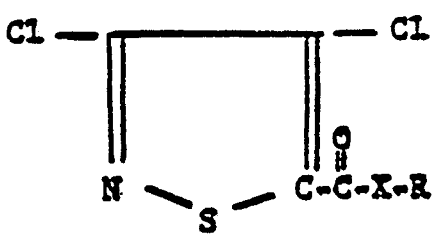

where X is oxygen, sulfur or - $\overset{H}{N}$ -; provided that when:

(i) X is oxygen, R is: naphthyl; phenyl; phenyl substituted with 1 to 2 halogens, halomethyl, 1 to 2 lower alkyl groups of 1 to 4 carbon atoms, 1 to 2 lower alkyl groups of 1 to 4 carbon atoms substituted with fluoro, 1 to 2 methoxy alkyl groups of 1 to 4 carbon atoms, 1 to 2 methoxy alkyl groups of 1 to 4 carbon atoms substituted with fluoro, 1 to 2 nitro groups or trifluoro; ethoxyphenyl; ethoxyphenyl substituted with chloro or methoxy; ethyl thiophenyl; ethyl thiophenyl substituted with chloro or methoxy; a polyethylene glycol bis where the number of ethylene oxide units is from 3 to 6; cyclohexyl; cyclohexyl monosubstituted with a lower alkyl group of 1 to 4 carbon atoms; or an akylene group of 2 to 4 carbon atoms;

(ii) X is - $\overset{H}{N}$ - , R is: phenyl; phenyl substituted with 1 to 2 nitro groups; or a pyridyl group and

(iii) X is sulfur, R is an alkylthioalkyl group in which each alkyl is of 1 to 4 carbon atoms.

7. The method of any one of claims 1 to 3 wherein the female fertile first plant parts are exposed to male fertile pollen from said second variety corn plant to provide pollinated first plant female parts, said pollinated female parts then maturing during the remainder of the growing season to provide a corn plant having hybrid corn seeds.

8. Hybric corn seeds prepared by the method as defined in claim 7.

9. Plant tissue of the corn plant prepared by the method as defined in claim 7.

10. A corn plant prepared by the method as defined in claim 7.